# EUROPÄISCHE PATENTANMELDUNG

(11) **EP 1 752 124 A1**
(43) Veröffentlichungstag der Anmeldung: **14.02.2007**
(21) Anmeldenummer: 05017584.3
(22) Anmeldetag: 12.08.2005
(51) Int. Cl.: A61H 35/00, A61N 1/44

(54) **Elektrolysefussbadgerät und Konverterspule**

(71) Anmelder: Body Detox AG, 8400 Winterthur (CH)
(72) Erfinder: Mosimann, François, 8400 Winterthur (CH); Artmann, Heribert, 93455 Traitsching - Höhhof (DE)
(74) Vertreter: Koepe, Gerd L.

(57) **Zusammenfassung**

Eine Konverterspule für ein Elektrolysefußbadgerät weist einen integrierten Speicherchip auf, wobei das Elektrolysefußbadgerät mit dem Speicherchip in Datenkommunikation steht.

## Beschreibung

Die Erfindung betrifft ein Elektrolysefußbadgerät und eine Konverterspule, die mit dem Elektrolysefußbadgerät betreibbar ist.

In der Medizin, in der Heilkunde und im Wellness-Bereich werden Elektrolysefußbäder als therapeutische Maßnahme zur Entschlackung und Entgiftung des menschlichen Körpers verordnet. Hierbei werden über die Haut der in eine Solelösung eingetauchten Füße toxische oder belastende aber auch körpereigene Stoffe ausgeschieden. Insbesondere werden bei der Anwendung des Elektrolysefußbads Schwermetalle, Harnsäure, Calcium, Magnesium, chlorierte Kohlenwasserstoffe, Biphenyle, usw. aus den Körper ausgeleitet.

Zur Durchführung eines Elektrolysefußbads werden herkömmliche Elektrolysefußbadgeräte verwendet, die eine Wanne und eine Konverterspule aufweisen. Die Wanne ist mit eventuell mit Zusätzen versehenem Wasser gefüllt, in das die Konverterspule eingetaucht ist. Die Konverterspule weist zwei Elektroden auf, die zueinander ein elektrisches Potential haben, auf Grund dessen eine Ionisierung des Wassers erzeugt wird. Sobald ein Patient seine Füße in das Wasser eintaucht, tritt über die von der Konverterspule erzeugte Ionisierung des Wassers die Wirkung des Elektrolysefußbads ein.

Die herkömmliche Konverterspule weist zwei schraubenwendelförmige zueinander konzentrisch angeordnete Elektroden auf, die aus einem Stahldraht gefertigt sind, dessen Material insbesondere einen hohen Nickel- und Chromanteil hat. Zum Betrieb der Konverterspule sind die Elektroden jeweils an einen Pol einer Spannungsquelle angeschlossen, so dass in dem Wasser ein Elektrolyseprozess stattfindet. Naturgemäß stellt sich im Betrieb an der Oberfläche der Elektroden ein Korrosionsprozess ein, der dazu führt, dass das Material der Elektroden ständig abgetragen wird. Dadurch werden die Elektrodendrähte im Betrieb stetig dünner, was bei ausreichender Betriebszeit der Konverterspule schließlich zum Bruch der Elektroden führt. Sind die Elektroden der Konverterspule gebrochen, so ist diese Konverterspule unbrauchbar und muss durch eine neue, unversehrte Konverterspule ausgetauscht werden. Passiert der Bruch der Elektroden der Konverterspule während einer Fußbadtherapie, so muss diese unterbrochen werden, bis das Fußbadgerät wieder in den ordentlichen Betriebszeitenzustand versetzt ist. Dies führt zu unerwünschten Verzögerungen bei der Fußbadtherapie.

Aufgabe der Erfindung ist es, eine intelligente Konverterspule und ein Elektrolysefußbadgerät dafür zu schaffen.

Die erfindungsgemäße Konverterspule für ein Elektrolysefußbadgerät weist einen integrierten Speicherchip auf. Der Speicherchip ist individuell mit Daten versehbar, die mit einem Elektrolysefußbadgerät kommunizierbar sind, in dem die Konverterspule betreibbar ist. Dadurch sind dem Elektrolysefußbadgerät Daten zur Verfügung stellbar, auf Grund dessen der Betrieb des Elektrolysefußbadgeräts manipuliert werden kann. Somit ist der Betrieb des Elektrolysefußbadgeräts in Abhängigkeit dieser Daten individuell auf die jeweilige Konverterspule abstimmbar.

Der Speicherchip kann einmalprogrammierbar eingerichtet sein. Bevorzugt ist der Speicherchip derart eingerichtet, in ihm eine Seriennummer und/oder eine Angabe zur Lebensdauer der Konverterspule beliebig oft einzugeben, permanent zu speichern, beliebig oft abzurufen und zu löschen. D.h., die Seriennummer und/oder die Angabe zur Lebensdauer der Konverterspule sind beliebig oft programmierbar und können jederzeit beliebig oft abgerufen werden. Dadurch können die Seriennummer und/oder die Angabe zur Lebensdauer der Konverterspule während deren Betrieb in dem Elektrolysefußbadgerät von diesem oder mittels eines anderen geeigneten Geräts den Erfordernissen des Betriebs oder sonstigen Erfordernissen entsprechend angepasst werden. Das Bereitstellen der Seriennummer, insbesondere der Seriennummer der Konverterspule, ermöglicht eine Identifikation der Konverterspule. Ferner ermöglicht das Bereitstellen der Angabe zur Lebensdauer der Konverterspule eine Anpassung des Betriebs der Konverterspule in Abhängigkeit zu dieser Angabe.

Das erfindungsgemäße Elektrolysefußbadgerät ist mit der erfindungsgemäßen Konverterspule betreibbar, wobei das Elektrolysefußbadgerät mit dem Speicherchip in Datenkommunikation steht. Durch das Elektrolysefußbadgerät sind Daten abrufbar, die von dem Speicherchip bereitgestellt sind, wobei der Betrieb des Elektrolysefußbadgeräts in Abhängigkeit dieser Daten angepasst werden kann. Dadurch, dass die Daten individuell auf die jeweilige Konverterspule abgestimmt sind, kann der Betrieb des Elektrolysefußbadgeräts auf diese jeweilige Konverterspule ausgerichtet werden.

Das Elektrolysefußbadgerät weist bevorzugt eine Steuerungseinheit zum Steuern des Betriebs der Konverterspule und eine Energieversorgungseinheit zur Versorgung der Steuerungseinheit und der Konverterspule mit Energie auf, wobei die Steuerungseinheit mit dem Speicherchip kommuniziert. Somit ist das Elektrolysefußbadgerät in die Lage versetzt, einerseits mittels der Steuerungseinheit die Datenkommunikation mit dem Speicherchip zu vollziehen und andererseits den Betrieb der Konverterspule in Abhängigkeit der von dem Speicherchip kommunizierten Daten zu steuern, wobei sowohl die Steuerungseinheit als auch die Konverterspule mittels der Energieversorgungseinheit mit Energie versorgt werden. Dadurch sind zum Betrieb des Elektrolysefußbadgeräts keine zusätzlichen Geräte beispielsweise zur Energieversorgung notwendig, wodurch das Elektrolysefußbadgerät kompakt im Aufbau ist und im Wesentlichen unabhängig von der Datenkommunikations- und der Energieversorgungsinfrastruktur von verschiedenen Betriebsorten ist.

Die Steuerungseinheit weist bevorzugt einen Prozessorchip auf, der eine Speicherchipschnittstelle zum Datenaustausch mit dem Speicherchip und eine Energieversorgungseinheitsschnittstelle zum Datenaustausch mit der Energieversorgungseinheit aufweist. Somit ist mittels des Prozessorchips via die Speicherchipschnittstelle der Datenaustausch mit dem Speicherchip und via die Energieversorgungseinheitsschnittstelle der Datenaustausch mit der Energieversorgungseinheit ermöglicht. Dadurch wird erreicht, dass der Prozessorchip als Steuerungsglied in der Steuerungseinheit wirkt, wobei der Datenaustausch sowohl mit dem Speicherchip als auch mit der Energieversorgungseinheit via die entsprechenden Schnittstellen gewährleistet ist.

Bevorzugt ist der Prozessorchip derart eingerichtet, via die Speicherchipschnittstelle auf dem Speicherchip gespeicherte Daten zu empfangen. Somit sind vorteilhaft diese Daten dem Prozessorchip zur Verarbeitung zur Verfügung gestellt.

Der Prozessorchip ist bevorzugt derart eingerichtet, in ihm ein Betriebsprofil beliebig oft einzugeben, permanent zu speichern, beliebig oft abzurufen, beliebig oft zu aktivieren und beliebig oft zu löschen. Dadurch ist es vorteilhaft ermöglicht, dass ein gewünschter Betrieb des Elektrolysefußbadgeräts in dem Prozessorchip bei entsprechender Ansteuerung der Konverterspule jederzeit verankert werden kann. Somit ist ein flexibler Betrieb des Elektrolysefußbadgeräts abgestimmt auf die Konverterspule und/oder einen Patienten ermöglicht.

Der Prozessorchip ist bevorzugt derart eingerichtet, die Energieversorgungseinheit via die Energieversorgungseinheitsschnittstelle zum Betrieb der Konverterspule in Abhängigkeit der von dem Speicherchip empfangenen Daten und des in dem Prozessorchip aktivierten Betriebsprofils anzusteuern. Somit ist beim Betrieb des Elektrolysefußbadgeräts die Energiezufuhr zu der Konverterspule steuerbar, indem die Energieversorgungseinheit von dem Prozessorchip via die Energieversorgungseinheitsschnittstelle derart angesteuert wird, dass die Energiezufuhr zu der Konverterspule auf das in dem Prozessorchip aktivierte Betriebsprofil abgestimmt ist. Dadurch ist es vorteilhaft ermöglicht, dass die Konverterspule entsprechend dem in dem Prozessorchip aktivierten Betriebsprofil eine über die Zeit variable Energiezufuhr empfängt. Diese kann darin bestehen, dass beispielsweise die elektrische Spannung, die an der Konverterspule anliegt, über die Zeit variiert, und/oder die Stromdurchflussrichtung an der Konverterspule wechselt.

Die Energieversorgungseinheit ist bevorzugt derart eingerichtet, die Konverterspule in Abhängigkeit der Ansteuerung durch die Steuerungseinheit mit Energie zu versorgen.

Somit ist beim Betrieb des Elektrolysefußbadgeräts die Energiezufuhr zu der Konverterspule so, wie sie von dem Prozessorchip durch Ansteuerung via die Energieversorgungseinheitsschnittstelle der Energieversorgungseinheit vorgegeben wird. Dadurch ist es vorteilhaft ermöglicht, dass die Konverterspule entsprechend dem in dem Prozessorchip aktivierten Betriebsprofil betrieben wird.

Bevorzugt weist der Prozessorchip eine Echtzeituhr auf. Mittels der Echtzeituhr ist beim Betrieb des Elektrolysefußbadgeräts jederzeit die von der Echtzeituhr angezeigte bzw. gemessene Echtzeit abrufbar. Bevorzugt ist die Echtzeituhr derart eingerichtet, die aktuelle Betriebsdauer der Konverterspule zu erfassen. Hierbei kann beispielsweise vorteilhaft mittels der Echtzeituhr die Lebensdauer der Konverterspule ermittelt werden.

Der Prozessorchip ist bevorzugt derart eingerichtet, die Abnutzung der Konverterspule durch den Betrieb in Abhängigkeit eines in dem Prozessorchip aktivierten Betriebsprofils und der von der Echtzeituhr erfassten Betriebsdauer zu ermitteln. Die Abnutzung der Konverterspule hängt insbesondere von der Stärke der elektrischen Spannung, die an der Konverterspule anliegt, und der Dauer ab, während deren diese Spannung an der Konverterspule anliegt. Dadurch, dass durch das in dem Prozessorchip aktivierte Betriebsprofil die elektrische Spannung vorgegeben ist, die an der Konverterspule anliegt, und die Dauer, während deren zum Betrieb der Konverterspule diese Spannung an dieser anliegt, von der Echtzeituhr bestimmbar ist, kann hiermit die Abnutzung der Konverterspule zuverlässig bestimmt werden. Somit ist vorteilhaft der Abnutzungsfortschritt der Konverterspule bekannt, so dass darauf reagierend bei entsprechend hoher Abnutzung und der Gefahr des Bruchs der Konverterspule während des Betriebs die abgenutzte Konverterspule gegen eine unversehrte Konverterspule ausgetauscht werden kann.

Der Prozessorchip ist bevorzugt derart eingerichtet, die Energieversorgungseinheit zum Betrieb der Konverterspule in Abhängigkeit eines in dem Prozessorchip aktivierten Betriebsprofils unter der Voraussetzung zu betreiben, dass die Lebensdauer der Konverterspule nicht abgelaufen ist und/oder die Seriennummer der Konverterspule von dem Prozessorchip identifiziert ist. Somit wird die Energieversorgungseinheit zum Betrieb der Konverterspule von dem Prozessorchip nur dann für die Energieversorgung der Konverterspule freigegeben, wenn die Konverterspule mittels ihrer von dem Speicherchip abrufbaren Seriennummer identifiziert ist und von dem Prozessorchip ermittelt ist, dass die Konverterspulenlebensdauer nicht abgelaufen ist, die über die Angaben auf dem Speicherchip der Konverterspule abrufbar ist. Dadurch wird vorteilhaft verhindert, dass die Konverterspule mit bereits abgelaufener Lebensdauer in das Elektrolysefußbadgerät eingesetzt betrieben wird. Mit der bevorzugten Einrichtung des Prozessorchips wird also unterbunden, dass, wenn die Lebensdauer der Konverterspule bereits abgelaufen ist und diese Konverterspule in dem Elektrolysefußbadgerät betrieben wird, die Konverterspule während des Betriebs bricht und die Elektrolysefußbadtherapie unterbrochen werden muss.

Bevorzugt ist der Prozessorchip derart eingerichtet, die um die Abnutzung der Konverterspule geminderte Lebensdauer der Konverterspule auf dem Speicherchip via die Speicherchipschnittstelle auszugeben. Somit ist auf dem Speicherchip eine stets aktualisierte Angabe zur Lebensdauer der Konverterspule abgespeichert und abrufbar. Dadurch ist vor jedem Betrieb der Konverterspule mittels dieser Angaben ermittelbar, ob die Lebensdauer der Konverterspule bereits abgelaufen ist oder ob die restliche, verbliebene Lebensdauer der Konverterspule ausreicht, um für einen neuen Betrieb geeignet zu sein bzw. dem Betrieb unter dem aktivierenden Betriebsprofil standhalten zu können ohne zu brechen.

Die Steuerungseinheit weist bevorzugt eine Bedieneinheit auf, die bevorzugt eine Eingabetastatur aufweist. Der Prozessorchip ist bevorzugt derart eingerichtet, dass er über die Eingabetastatur manipulierbar ist. Dadurch können Daten über die Eingabetastatur in den Prozessorchip eingegeben werden, insbesondere Daten über den gewünschten Betrieb des Elektrolysefußbadgeräts.

Bevorzugt ist der Prozessorchip derart eingerichtet, in ihm ein Betriebsprofil über die Eingabetastatur abzurufen und zu aktivieren. Dadurch kann mittels der Eingabetastatur ein gewünschtes Betriebsprofil aus der Vielzahl von Betriebsprofilen, die in dem Prozessorchip abgespeichert sind, abgerufen und aktiviert werden. Somit ist vorteilhaft ein vielseitiger Betrieb des Elektrolysefußbadgeräts ermöglicht.

Ferner weist die Bedieneinheit bevorzugt einen Anzeigeschirm auf. Dadurch ist es ermöglicht, dass Daten aus dem Prozessorchip und/oder dem Speicherchip dem Patienten angezeigt werden können. Somit ist mittels des Anzeigeschirms in Kombination mit der Eingabetastatur eine interaktive Bedienung des Elektrolysefußbadgeräts ermöglicht.

Bevorzugt ist der Anzeigeschirm derart eingerichtet, die Seriennummer der Konverterspule und/oder die Lebensdauer der Konverterspule anzuzeigen. Da die Seriennummer der Konverterspule und/oder die Lebensdauer der Konverterspule auf dem Speicherchip der Konverterspule von dem Prozessorchip abrufbar zur Verfügung stehen, ist eine Anzeige dieser Angaben auf dem Anzeigeschirm ermöglicht. Somit sind die auf dem Speicherchip abgespeicherten Daten von dem Patienten oder einer anderen Person via den Anzeigeschirm einsehbar.

Bevorzugt ist der Anzeigeschirm derart eingerichtet, eine Menüstruktur zur Auswahl und zur Aktivierung eines Betriebsprofils in dem Prozessorchip anzuzeigen. Dadurch ist es ermöglicht, dass die Auswahl und die Aktivierung eines gewünschten Betriebsprofils aus der Vielzahl von den in dem Prozessorchip abgespeicherten Betriebsprofilen menügesteuert interaktiv getätigt werden kann. Somit ist die Bedienung des Elektrolysefußbadgeräts bequem und transparent.

Der Anzeigeschirm ist derart eingerichtet, die aktuelle Betriebsdauer der Konverterspule anzuzeigen. Dadurch ist die Angabe zur aktuellen Betriebsdauer der Konverterspule dem Patienten oder einer anderen Person zugänglich gemacht. Somit ist es dem Patienten oder der anderen Person ermöglicht, anhand der Angabe zur aktuellen Betriebsdauer der Konverterspule abzuschätzen, wie lange eine eben laufende Behandlung durch das Elektrolysefußbadgerät noch andauert.

Ferner ist der Anzeigeschirm bevorzugt derart eingerichtet, ein in dem Prozessorchip aktiviertes Betriebsprofil anzuzeigen. Dadurch sind vorteilhaft dem Patienten oder der anderen Person diese Angaben zugänglich gemacht.

Außerdem ist der Anzeigeschirm bevorzugt derart eingerichtet, einen Alarm bei abgelaufener Lebensdauer der Konverterspule anzuzeigen. Hierbei wird der Umstand, dass die Lebensdauer der Konverterspule abgelaufen ist, die in das Elektrolysefußbadgerät zum Betreiben desselben eingesetzt ist, dem Patienten oder der anderen Person kenntlich gemacht. Vorteilhaft ist der Patient oder die andere Person darüber informiert, dass das Elektrolysefußbadgerät mit der Konverterspule nicht mehr betreibbar ist. Als Folge davon kann die Konverterspule mit abgelaufener Lebensdauer durch eine neue Konverterspule mit noch nicht abgelaufener Lebensdauer ausgetauscht werden.

Das Elektrolysefußbadgerät ist bevorzugt mit einer lichttherapeutischen Einrichtung versehen. Dadurch ergibt sich vorteilhaft zusätzlich die Möglichkeit des Einsatzes einer Lichttherapie, wodurch die Therapiewirkung des Fußbades verbessert werden kann. Bevorzugt ist der Prozessorchip derart eingerichtet, die Energieversorgungseinheit zum Betrieb der lichttherapeutischen Einrichtung anzusteuern. Dadurch ist es vorteilhaft ermöglicht, die Lichttherapie mittels der lichttherapeutischen Einrichtung von dem Prozessorchip gesteuert durchzuführen, insbesondere in Abstimmung mit dem Betrieb der Konverterspule.

Bevorzugt ist die Energieversorgungseinheit derart eingerichtet, die lichttherapeutische Einrichtung in Abhängigkeit der Ansteuerung durch die Steuerungseinheit mit Energie zu versorgen. Somit ist beim Betrieb des Elektrolysefußbadgeräts die Energiezufuhr zu der lichttherapeutischen Einrichtung so, wie sie von dem Prozessorchip durch Ansteuerung via die Energieversorgungseinheitsschnittstelle der Energieversorgungseinheit vorgegeben wird. Dadurch ist es vorteilhaft ermöglicht, dass die lichttherapeutische Einrichtung von dem Prozessorchip gesteuert betrieben wird.

Ferner ist bevorzugt das Elektrolysefußbadgerät mit einer magnettherapeutischen Einrichtung ausgestattet. Die magnettherapeutische Einrichtung kann zusammen mit der lichttherapeutischen Einrichtung betrieben oder ohne diese zur Verbesserung des Therapieeffekts des Elektrolysefußbads eingesetzt werden. Der Prozessorchip ist bevorzugt derart eingerichtet, die Energieversorgungseinheit zum Betrieb der magnettherapeutischen Einrichtung anzusteuern. Dadurch ist es vorteilhaft ermöglicht, die Magnettherapie mittels der magnettherapeutischen Einrichtung von dem Prozessorchip gesteuert durchzuführen, insbesondere in Abstimmung mit dem Betrieb der Konverterspule und/oder der lichttherapeutischen Einrichtung.

Bevorzugt ist die Energieversorgungseinheit derart eingerichtet, die magnettherapeutische Einrichtung in Abhängigkeit der Ansteuerung durch die Steuerungseinheit mit Energie zu versorgen. Somit ist beim Betrieb des Elektrolysefußbadgeräts die Energiezufuhr zu der magnettherapeutischen Einrichtung so, wie sie von dem Prozessorchip durch Ansteuerung via die Energieversorgungseinheitsschnittstelle der Energieversorgungseinheit vorgegeben wird. Dadurch ist es vorteilhaft ermöglicht, dass die magnettherapeutische Einrichtung von dem Prozessorchip gesteuert betrieben wird.

Im Folgenden wird die Erfindung anhand bevorzugter Ausführungsformen mit Bezugnahme auf die Zeichnung erläutert. In der Zeichnung zeigen:
Figur 1 eine perspektivische Ansicht einer ersten Ausführungsform eines erfindungsgemäßen Elektrolysefußbadgeräts mit einer erfindungsgemäßen Konverterspule,
Figur 2 eine Explosionsdarstellung der erfindungsgemäßen Konverterspule aus Figur 1,
Figur 3 eine perspektivische Ansicht einer zweiten Ausführungsform eines erfindungsgemäßen Elektrolysefußbadgeräts mit einer erfindungsgemäßen Konverterspule und
Figur 4 ein Blockschaltbild der ersten und zweiten Ausführungsform.

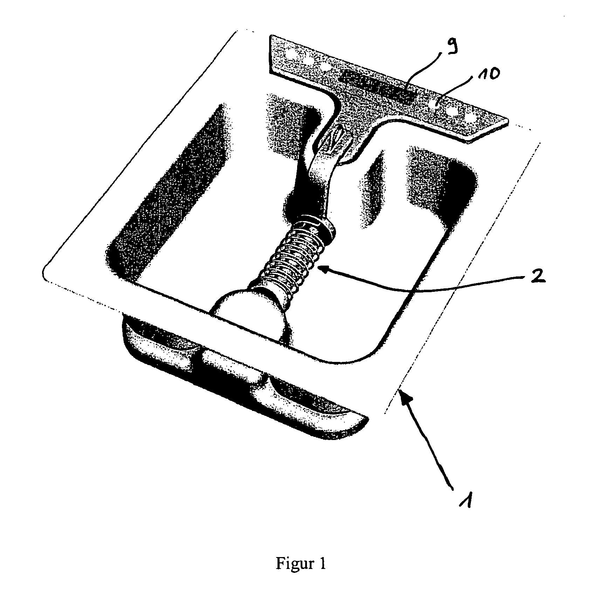

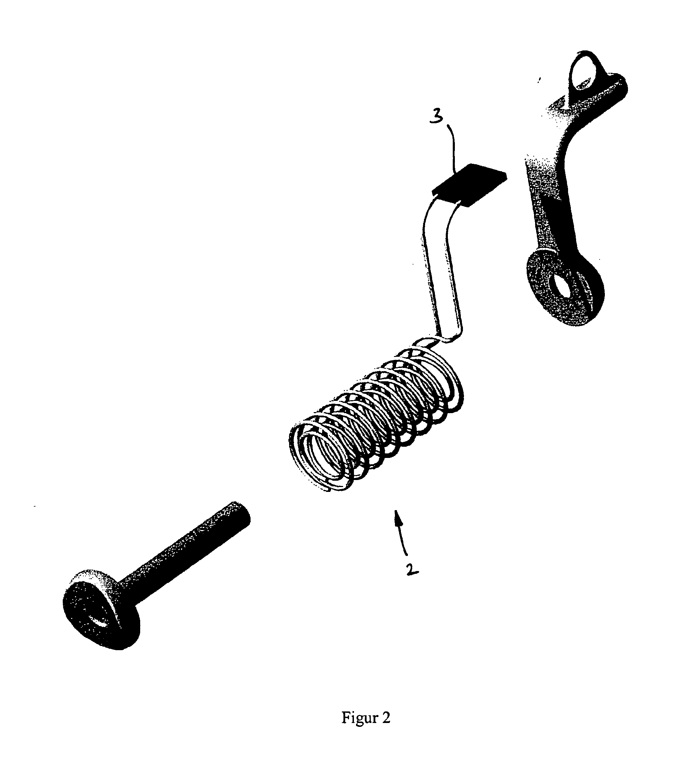

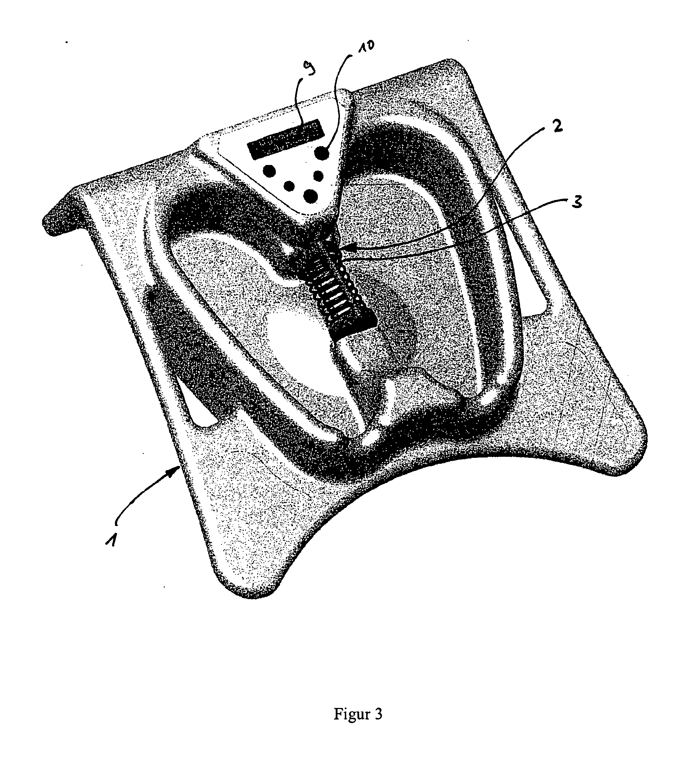

Wie aus Figuren 1 bis 3 ersichtlich ist in ein erfindungsgemäßes Elektrolysefußbadgerät 1 eine erfindungsgemäße Konverterspule 2 eingesetzt. Die Konverterspule 2 weist einen Speicherchip 3 auf. Das Elektrolysefußbadgerät 1 weist an seiner Oberseite einen Anzeigeschirm 9 und eine Eingabetastatur 10 auf, wobei der Anzeigeschirm 9 von oben gut einsehbar und die Eingabetastatur 10 oben gut zugänglich angebracht sind.

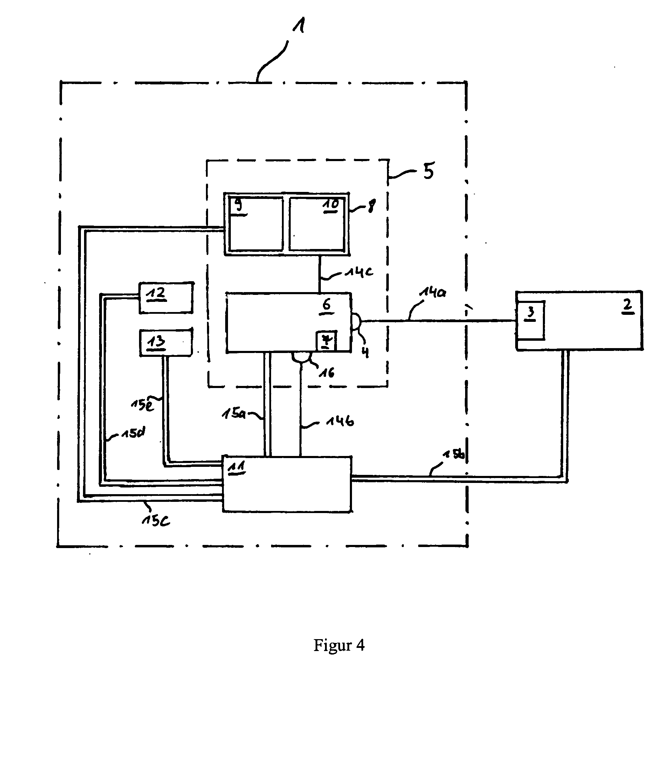

In Figur 4 ist ein Blockschaltbild gezeigt, in dem prinzipiell der Aufbau des Elektrolysefußbadgeräts 1 und der Konverterspule 2 dargestellt ist. Wie es aus Figur 4 ersichtlich ist, weist das Elektrolysefußbadgerät 1 eine Steuerungseinheit 5 auf, in der ein Prozessorchip 6 und eine Bedieneinheit 8 vorgesehen sind. Der Prozessorchip 6 weist eine Speicherchipschnittstelle 4 auf, die via eine Informationsleitung 14a mit dem Speicherchip 3 der Konverterspule 2 zum Datenaustausch zwischen dem Prozessorchip 6 und dem Speicherchip 3 zusammenwirkt. Ferner weist das Elektrolysefußbadgerät 1 eine Energieversorgungseinheit 11 auf. Ein Datenaustausch zwischen dem Prozessorchip 6 und der Energieversorgungseinheit 11 wird mittels einer der Prozessorchip 6 aufweisende Energieversorgungseinheitsschnittstelle 16 bewerkstelligt, die via eine Informationsleitung 14b mit der Energieversorgungseinheit 11 zusammenwirkt. Die Energieversorgungseinheit 11 ist mit dem Prozessor 6 via der Energieleitung 15a und mit der Konverterspule 2 via der Energieleitung 15b zur Energieversorgung des Prozessors 6 bzw. der Konverterspule 2 verbunden.

Der Prozessor weist eine Echtzeituhr 7 auf, mittels der die aktuelle Betriebsdauer der Konverterspule 2 von dem Prozessor 6 erfassbar ist.

Die Bedieneinheit 8 weist den Anzeigeschirm 9 und die Eingabetastatur 10 auf, wobei der Datenaustausch zwischen der Bedieneinheit 8 und dem Prozessorchip 6 via die Informationsleitung 14c bewerkstelligt wird.

Das Elektrolysefußbadgerät 1 weist außerdem eine lichttherapeutische Einrichtung 12 und eine magnettherapeutische Einrichtung 13 auf, wobei diese beiden Einrichtungen 12, 13 für deren Energieversorgung von der Energieversorgungseinheit 11 mit dieser via Energieversorgungsleitungen 15d, 15e verbunden sind. Ferner ist zur Energieversorgung der Bedieneinheit 8 diese via eine Energieleitung 15c mit der Energieversorgungseinheit 11 verbunden.

In dem Speicherchip 3 sind eine Seriennummer der Konverterspule 2 und deren aktuelle noch verbleibende Lebensdauer eingegeben und permanent gespeichert. Via der Informationsleitung 14a und mittels der Speicherchipschnittstelle 4 sind diese Daten durch den Prozessorchip 6 von dem Speicherchip 3 abrufbar. In dem Prozessorchip 6 sind eine Vielzahl von Betriebsprofilen einprogrammiert, die jeweils einen Datensatz umfassen, der einer bestimmten Betriebsart der Konverterspule zugeordnet ist. Durch ein bestimmtes Betriebsprofil wird die Stärke, der zeitliche Verlauf und die Richtung der elektrischen Spannung, die an der Konverterspule anliegen soll, und die Dauer dieser Spannungsbeaufschlagung festgelegt.

In dem Prozessorchip 6 ist ferner zur Aktivierung eines bestimmten Betriebsprofils eine Menüstruktur programmiert, die einerseits über Datenaustausch via die Informationsleitung 14c von dem Prozessorchip 6 zu der Bedieneinheit 8 von dem Anzeigeschirm 9 darstellbar und andererseits über Datenaustausch via die Informationsleitung 14c von der Bedieneinheit 8 zu dem Prozessorchip 6 von der Eingabetastatur 10 verwendbar ist. Somit kann mittels des Anzeigeschirms 9 und der Eingabetastatur 10 unter Heranziehen der Menüstruktur der Prozessorchip 6 derart manipuliert werden, so dass ein bestimmtes Betriebsprofil aus der Vielzahl von in dem Prozessorchip 6 abgespeicherten Betriebsprofilen aufrufbar und aktivierbar ist.

Der Prozessorchip 6 ist derart programmiert, dass eine Aktivierung eines Betriebsprofils erst dann freigegeben wird, wenn die von dem Speicherchip 3 empfangenen Daten ausgewertet worden sind und diese Auswertung ergibt, dass der Beginn des Betriebs der Konverterspule 2 sinnvoll möglich ist, d.h., dass während des Betriebs nicht mit einem Bruch der Konverterspule 2 gerechnet zu werden braucht. Im Rahmen dieser Auswertung wird die Konverterspule 2 mittels der Seriennummer identifiziert, wobei die Seriennummer entsprechend vordefinierten Voraussetzungen über das Format und den Zahlenbereich zu genügen hat, und die Betriebssicherheit der Konverterspule 2 bestimmt, wobei die zu erwartende Abnutzung der Konverterspule 2 bei deren Betrieb gemäß dem ausgewählten Betriebsprofil der aktuell tatsächlich zur Verfügung stehenden Lebensdauer der Konverterspule 2 angerechnet wird. Ist von dem Prozessorchip 6 die Seriennummer als eine solche identifiziert und ist das Ergebnis der Ermittlung positiv, dass während des Betriebs der Konverterspule 2 unter dem ausgewählten Betriebsprofil die Lebensdauer der Konverterspule 2 nicht abläuft, so wird in dem Prozessorchip 6 die Aktivierung des ausgewählten Betriebsprofils freigegeben. Ansonsten ist die Aktivierung eines ausgewählten Betriebsprofils gesperrt. Die Aktivierung des ausgewählten Betriebsprofils ist mittels des Anzeigeschirms 9 und der Eingabetastatur 10 unter Zuhilfenahme einer von dem Prozessorchip 6 zur Verfügung gestellten Menüstruktur zu bewerkstelligen.

Sobald das ausgewählte Betriebsprofil aktiviert ist, wird die Energieversorgungseinheit 11 von dem Prozessorchip 6 mittels der Energieversorgungseinheitsschnittstelle 16 via die Informationsleitung 14b entsprechend dem aktivierten Betriebsprofil angesteuert. Die Ansteuerung erfolgt derart, dass an der Konverterspule 2 mittels der Energieversorgungseinheit 11 via die Energieleitung 15b ein Spannungsprofil über eine entsprechende Dauer gemäß dem ausgewählten Betriebsprofil anliegt.

Mittels der Echtzeituhr 7 steht im Prozessorchip 6 die Echtzeit zur Verfügung. Der Prozessorchip 6 ist derart programmiert, dass von der Echtzeituhr 7 am Beginn und am Ende der Dauer des Betriebs der Konverterspule 2 die Echtzeit abgerufen wird, woraus die tatsächliche Betriebsdauer der Konverterspule 2 ermittelt wird. Ferner ist der Prozessorchip 6 derart programmiert, dass nach Beendigung des Betriebs der Konverterspule 2 unter Berücksichtigung der tatsächlichen Betriebsdauer und des aktivierten Betriebsprofils die Abnutzung der Konverterspule 2 aufgrund des absolvierten Betriebs abgeschätzt und die ursprünglich abgespeicherte Angabe zur Lebensdauer der Konverterspule 2 um einen entsprechenden Betrag reduziert wird. Anschließend wird von dem Prozessorchip 6 über die Speicherchipschnittstelle 4 via die Informationsleitung 14a die auf dem Speicherchip 3 abgespeicherte Angabe zur Lebensdauer der Konverterspule 2 gelöscht und durch die Angabe über die reduzierte Lebensdauer ersetzt, so dass wieder die tatsächliche aktuelle Lebensdauer der Konverterspule 2 auf dem Speicherchip 3 abgespeichert ist.

Ferner ist der Prozessorchip 6 derart programmiert, dass, wenn die Analyse der von dem Speicherchip abgerufenen Daten negativ ist, von dem Prozessorchip 6 via die Informationsleitung 14c die Steuerungseinheit 5 derart angesteuert wird, dass der Anzeigeschirm 9 ein Alarmsignal anzeigt. Von dem Prozessorchip 6 wird ein derartiges Alarmsignal erzeugt, wenn die auf dem Speicherchip 3 abgespeicherte Seriennummer nicht identifizierbar ist und/oder die auf dem Speicherchip 3 abgespeicherte Lebensdauer der Konverterspule 2 nicht ausreichend ist, um bei Ablauf des ausgewählten Betriebsprofils nicht unterschritten zu werden.

Dadurch, dass auf dem Speicherchip 3 stets die aktuelle, tatsächlich zur Verfügung stehende Lebensdauer der Konverterspule 2 von dem Prozessorchip 6 abrufbar ist, wird ein Betrieb des Elektrolysefußbadgeräts 1 mit der Konverterspule 2 ohne Ablauf der Lebensdauer derselben ermöglicht. Die auf dem Speicherchip 3 hinterlegten Angaben zur Lebensdauer der Konverterspule 2 sind derart bestimmt, dass sie eine Aussage über die Wahrscheinlichkeit des Auftretens eines Bruchs der Konverterspule 2 während deren Betriebs aufgrund von Korrosion zulassen. Ein Ablaufen der Lebensdauer der Konverterspule 2 bedeutet, dass die Wahrscheinlichkeit, dass während des Betriebs der Konverterspule 2 ein Bruch derselben auftritt, als zu hoch angesehen wird. Im Gegensatz dazu, ist die Lebensdauer der Konverterspule 2 noch nicht abgelaufen, wird die Wahrscheinlichkeit des Auftretens eines Bruchs der Konverterspule 2 während des Betriebs derselben als gering oder als noch vertretbar angesehen.

Der Prozessorchip 6 ist derart programmiert, dass das ausgewählte Betriebsprofil nur dann aktivierbar ist, wenn von dem Prozessorchip 6 erkannt wird, dass die auf dem Speicherchip 3 abgespeicherte Lebensdauer noch nicht abgelaufen ist oder zur Beendigung des Betriebs aufgrund des ausgewählten Betriebsprofils bei Vermeidung eines Bruchs der Konverterspule 2 während deren Betriebs noch ausreichend ist. Somit ist die Wahrscheinlichkeit gering, dass die Konverterspule während des Betriebs bricht.

Die in dem Prozessorchip 6 bereitgestellten Betriebsprofile weisen optional auch einen Fahrplan zum Betrieb sowohl der lichttherapeutischen Einrichtung 12 als auch der magnettherapeutischen Einrichtung 13 auf. Ist in dem Prozessor 6 ein entsprechendes Betriebsprofil aktiviert bei dem der Betrieb der lichttherapeutischen Einrichtung 12 und der magnettherapeutischen Einrichtung 13 vorgesehen ist, so wird die Energieversorgungseinheit 11 von dem Prozessorchip 6 über die Energieversorgungseinheitsschnittstelle 16 via die Informationsleitung 14b so angesteuert, so dass die lichttherapeutische Einrichtung 12 und die magnettherapeutische Einrichtung 13 in Abhängigkeit von dieser Steuerung und somit entsprechend dem aktivierten Betriebsprofil von der Energieversorgungseinheit 11 mit Energie versorgt werden.

In den entsprechenden Betriebsprofilen ist der Betrieb der lichttherapeutischen Einrichtung 12 derart vorgesehen, dass während der Therapie die lichttherapeutische Einrichtung 12 ein Dauerlicht bei konstanter Lichtstärke emittiert oder blinkt. In den entsprechenden Betriebsprofilen ist der Betrieb der magnettherapeutischen Einrichtung 13 derart vorgesehen, dass die magnettherapeutische Einrichtung 13 ein Magnetfeld mit konstanter Feldstärke oder mit wechselnder Feldstärke erzeugt.

Entsprechend der Berücksichtigung der lichttherapeutischen Einrichtung 12 und der magnettherapeutischen Einrichtung 13 in einem entsprechenden Betriebsprofil wird die Konverterspule 2 zusammen mit der lichttherapeutischen Einrichtung 12 und/oder der magnettherapeutischen Einrichtung 13 betrieben, wodurch der therapeutische Effekt des Elektrolysefußbadgeräts erhöht werden kann.

### Bezugszeichenliste

- 1: Elektrolysefußbadgerät
- 2: Konverterspule
- 3: Speicherchip
- 4: Speicherchipschnittstelle
- 5: Steuerungseinheit
- 6: Prozessorchip
- 7: Echtzeituhr
- 8: Bedieneinheit
- 9: Anzeigeschirm
- 10: Eingabetastatur
- 11: Energieversorgungseinheit
- 12: lichttherapeutische Einrichtung
- 13: magnettherapeutische Einrichtung
- 14: Informationsleitung
- 15: Energieleitung
- 16: Energieversorgungseinheitsschnittstelle

## Patentansprüche

1. Konverterspule für ein Elektrolysefußbadgerät (1), mit integriertem Speicherchip (3).

2. Konverterspule gemäß Anspruch 1, wobei der Speicherchip (3) derart eingerichtet ist, in ihm eine Seriennummer beliebig oft einzugeben, permanent zu speichern, beliebig oft abzurufen und beliebig oft zu löschen.

3. Konverterspule gemäß Anspruch 1 oder 2, wobei der Speicherchip (3) derart eingerichtet ist, in ihm eine Angabe zur Lebensdauer der Konverterspule (2) beliebig oft einzugeben, permanent zu speichern, beliebig oft abzurufen und beliebig oft zu löschen.

4. Elektrolysefußbadgerät für eine Konverterspule (2) gemäß einem der Ansprüche 1 bis 3, wobei das Elektrolysefußbadgerät (1) mit dem Speicherchip (3) in Datenkommunikation steht.

5. Elektrolysefußbadgerät gemäß Anspruch 4, mit einer Steuerungseinheit (5) zum Steuern des Betriebs der Konverterspule (2) und mit einer Energieversorgungseinheit (11) zur Versorgung der Steuerungseinheit (5) und der Konverterspule (2) mit Energie, wobei die Steuerungseinheit (5) mit dem Speicherchip (3) kommuniziert.

6. Elektrolysefußbadgerät gemäß Anspruch 5, wobei die Steuerungseinheit (5) einen Prozessorchip (6) mit einer Speicherchipschnittstelle (4) zum Datenaustausch mit dem Speicherchip (3) und mit einer Energieversorgungseinheitsschnittstelle (16) zum Datenaustausch mit der Energieversorgungseinheit (11) aufweist.

7. Elektrolysefußbadgerät gemäß Anspruch 6, wobei der Prozessorchip (6) derart eingerichtet ist, via die Speicherchipschnittstelle (4) auf dem Speicherchip (3) gespeicherte Daten zu empfangen.

8. Elektrolysefußbadgerät gemäß Anspruch 6 oder 7, wobei der Prozessorchip (6) derart eingerichtet ist, in ihm ein Betriebsprofil beliebig oft einzugeben, permanent zu speichern, beliebig oft abzurufen, beliebig oft zu aktivieren und beliebig oft zu löschen.

9. Elektrolysefußbadgerät gemäß Anspruch 6 oder 8, wobei der Prozessorchip (6) derart eingerichtet ist, die Energieversorgungseinheit (11) via die Energieversorgungseinheitsschnittsstelle (16) zum Betrieb der Konverterspule (2) in Abhängigkeit der von dem Speicherchip (3) empfangenen Daten und des in dem Prozessorchip (6) aktivierten Betriebsprofils anzusteuern.

10. Elektrolysefußbadgerät gemäß Anspruch 9, wobei die Energieversorgungseinheit (11) derart eingerichtet ist, die Konverterspule (2) in Abhängigkeit der Ansteuerung durch die Steuerungseinheit (5) mit Energie zu versorgen.

11. Elektrolysefußbadgerät gemäß einem der Ansprüche 6 bis 10, wobei der Prozessorchip (6) eine Echtzeituhr (7) aufweist.

12. Elektrolysefußbadgerät gemäß Anspruch 11, wobei die Echtzeituhr (7) derart eingerichtet ist, die aktuelle Betriebsdauer der Konverterspule (2) zu erfassen.

13. Elektrolysefußbadgerät gemäß Anspruch 12, wobei der Prozessorchip (6) derart eingerichtet ist, die Abnutzung der Konverterspule (2) durch den Betrieb in Abhängigkeit eines in dem Prozessorchip (6) aktivierten Betriebsprofils und der von der Echtzeituhr (7) erfassten Betriebsdauer zu ermitteln.

14. Elektrolysefußbadgerät gemäß Anspruch 13, wobei der Prozessorchip (6) derart eingerichtet ist, die Energieversorgungseinheit (11) zum Betrieb der Konverterspule (2) in Abhängigkeit eines in dem Prozessorchip (6) aktivierten Betriebsprofils unter der Voraussetzung zu betreiben, dass die Lebensdauer der Konverterspule (2) nicht abgelaufen ist und /oder die Seriennummer der Konverterspule (2) von dem Prozessorchip (6) identifiziert ist.

15. Elektrolysefußbadgerät gemäß Anspruch 13 oder 14, wobei der Prozessorchip (6) derart eingerichtet ist, die um die Abnutzung der Konverterspule (2) geminderte Lebensdauer der Konverterspule (2) auf dem Speicherchip (3) via die Speicherchipschnittstelle (4) auszugeben.

16. Elektrolysefußbadgerät gemäß einem der Ansprüche 5 bis 15, wobei die Steuerungseinheit (5) eine Bedieneinheit (8) aufweist.

17. Elektrolysefußbadgerät gemäß Anspruch 16, wobei die Bedieneinheit (8) eine Eingabetastatur (10) aufweist.

18. Elektrolysefußbadgerät gemäß Anspruch 17, wobei der Prozessorchip (6) derart eingerichtet ist, über die Eingabetastatur (10) manipulierbar zu sein.

19. Elektrolysefußbadgerät gemäß Anspruch 18, wobei der Prozessorchip (6) derart eingerichtet ist, in ihm Betriebsprofile über die Eingabetastatur (10) abzurufen und zu aktivieren.

20. Elektrolysefußbadgerät gemäß einem der Ansprüche 16 bis 19, wobei die Bedieneinheit (8) einen Anzeigeschirm (9) aufweist.

21. Elektrolysefußbadgerät gemäß Anspruch 20, wobei der Anzeigeschirm (9) derart eingerichtet ist, die Seriennummer der Konverterspule (2) und/oder die Lebensdauer der Konverterspule (2) anzuzeigen.

22. Elektrolysefußbadgerät gemäß 20 oder 21, wobei der Anzeigeschirm (9) derart eingerichtet ist, eine Menüstruktur zur Auswahl und zur Aktivierung eines Betriebsprofils in dem Prozessorchip (6) anzuzeigen.

23. Elektrolysefußbadgerät gemäß einem der Ansprüche 20 bis 22, wobei der Anzeigeschirm (9) derart eingerichtet ist, die aktuelle Betriebsdauer der Konverterspule (2) anzuzeigen.

24. Elektrolysefußbadgerät gemäß einem der Ansprüche 20 bis 23, wobei der Anzeigeschirm (9) derart eingerichtet ist, ein in dem Prozessorchip (6) aktiviertes Betriebsprofil anzuzeigen.

25. Elektrolysefußbadgerät gemäß einem der Ansprüche 20 bis 24, wobei der Anzeigeschirm (9) derart eingerichtet ist, einen Alarm bei abgelaufener Lebensdauer der Konverterspule (2) anzuzeigen.

26. Elektrolysefußbadgerät gemäß einem der Ansprüche 6 bis 25, mit einer lichttherapeutischen Einrichtung (12).

27. Elektrolysefußbadgerät gemäß Anspruch 26, wobei der Prozessorchip (6) derart eingerichtet ist, die Energieversorgungseinheit (11) zum Betrieb der lichttherapeutischen Einrichtung (12) anzusteuern.

28. Elektrolysefußbadgerät gemäß Anspruch 27, wobei die Energieversorgungseinheit (11) derart eingerichtet ist, die lichttherapeutische Einrichtung (12) in Abhängigkeit der Ansteuerung durch die Steuerungseinheit (5) mit Energie zu versorgen.

29. Elektrolysefußbadgerät gemäß einem der Ansprüche 6 bis 28, mit einer magnettherapeutischen Einrichtung (13).

30. Elektrolysefußbadgerät gemäß Anspruch 29, wobei der Prozessorchip (6) derart eingerichtet ist, die Energieversorgungseinheit (11) zum Betrieb der magnettherapeutischen Einrichtung (13) anzusteuern.

31. Elektrolysefußbadgerät gemäß Anspruch 30, wobei die Energieversorgungseinheit (11) derart eingerichtet ist, die magnettherapeutische Einrichtung (13) in Abhängigkeit der Ansteuerung durch den Prozessorchip (6) mit Energie zu versorgen.
